# EUROPEAN PATENT SPECIFICATION

(11) **EP 3 755 195 B1**
(45) Date of publication and mention of the grant of the patent: **03.12.2025**
(21) Application number: 19712303.7
(22) Date of filing: 22.02.2019
(51) Int. Cl.: A61B 1/00, A61B 1/12, A61B 90/70

(54) **SUPPORT DEVICE FOR ENDOSCOPIC INSTRUMENTS TO BE SUBJECTED TO TREATMENT**
HALTEVORRICHTUNG ZUM HALTEN EINES ENDOSKOPISCHEN INSTRUMENTS
DISPOSITIF PORTEUR POUR SUPPORTER UN INSTRUMENT ENDOSCOPIQUE

(30) Priority: 23.02.2018 IT 201800003002
(43) Date of publication of application: 30.12.2020
(73) Proprietor: Steelco S.p.A., 31039 Riese Pio X (IT)
(72) Inventor: CALZAVARA, Francesco, 31033 CASTELFRANCO VENETO (IT)
(74) Representative: Petraz, Gilberto Luigi
(86) International application number: PCT/IT2019/050038
(87) International publication number: WO 2019/162979

(56) References cited:
- WO-A1-2015/107801
- WO-A1-2017/187919
- WO-A2-2017/202830
- JP-A- H03 280 925
- JP-A- H04 314 418

## Description

### FIELD OF THE INVENTION

Embodiments described here concern a support device for endoscopic instruments to be subjected to treatment, for example a rack for washing endoscopes, and in particular for duodenoscopes.

### BACKGROUND OF THE INVENTION

It is known that flexible endoscopes are commonly used both for diagnostic procedures and also for therapeutic purposes. For example, endoscopes can be used to diagnose and/or treat medical conditions that affect the lungs, esophagus, stomach, small intestine, biliary tract, pancreas or colon.

It is also known that endoscopes are instruments that are reused many times on different patients, and therefore it is necessary to perform a high level of disinfection to eliminate therefrom any possible infectious loads that could contaminate a patient.

In the absence of adequate disinfection, in fact, in patients undergoing endoscopy, infections can occur that can be the result of the patient's own flora, or the result of microbes introduced by the endoscope or its accessories.

Endoscopes generally consist of one or more materials that can be chemically sensitive or which can degrade over time and with use. In addition, due to the complex shape and the components and adhesives used that are sensitive to heat, endoscopes cannot be sterilized in an autoclave by steam.

Consequently, most modern endoscopes cannot be sterilized, indicating with this term the complete removal or destruction of all microbial cells or spores.

Endoscopes are therefore subjected to disinfection, meaning by this term the removal or elimination of most of the microorganisms, but not all. High level disinfection is considered adequate for the treatment of endoscopes since it removes or eliminates microorganisms that are more likely to cause diseases.

Many treatment systems use a combination of chemical agents and moderate heat to achieve a high level of disinfection, but these may not be sufficient, for example in the case of treating duodenoscopes that have a complex structure.

In fact, duodenoscopes have a specific structure in correspondence with their distal end, usable to modify the angle of the catheters, called forceps elevator, or fin, and a channel associated therewith. The forceps elevator can be rotated between a lowered position, in which it is substantially aligned with a longitudinal axis of the endoscope, to allow it to slide in the human body, and a raised position, in which it is rotated with respect to said axis and protrudes in a transverse direction.

This constructive complexity makes disinfection more difficult, and this increased difficulty has contributed to a higher risk of infection compared to the risk associated with other types of endoscopes (for example gastroscopes).

In recent years there have been several cases of patients exposed to multi-drug resistant organisms (MDROs), for example carbapenem-resistant enterobacteria (CRE) due to contaminated duedonoscopes. The cases of CRE transmission have been associated with the use of contaminated duodenoscopes used to perform endoscopic retrograde cholangiopancreatography (ERCP), a procedure that is performed even half a million times each year.

However, CREs are only the infectious agents that have been associated most recently with contaminated endoscopes; many other diseases that can be transmitted through endoscopic procedures are also known.

In order to solve the problem, regulations and guidelines have been defined which provide a specific washing and disinfection treatment for duodenoscopes, and in particular for the distal end thereof which is provided with the elevator.

Because of the elaborate and non-linear shape of the distal end, due to the presence of a forceps elevator, in fact, bacteria and microorganisms can settle in the interstices that are formed and, if they are not removed properly, they can be extremely harmful to patients. In particular, the international guidelines commonly adopted for the sanitization of duodenoscopes expressly require that the distal end in question is subjected to specific, dedicated and localized treatment, to overcome the criticalities set forth above.

These guidelines require manual pre-washing of the distal end, using brushes or cleaners, and subsequent washing by suitable treatment machines.

For example, treatment machines are known which provide to treat the endoscopic instrument by immersion. Immersion treatment machines, however, require to use large quantities of treatment liquid and in any case do not guarantee an optimum level of disinfection in the treatment of the distal end of a duodenoscope.

Apparatuses for washing endoscopes provided with support devices are also known, in which the articles to be subjected to washing and disinfection are positioned and suitably clamped, to be then hit by jets of washing liquid in suitable treatment machines. However, even these known solutions do not guarantee a specific cleaning of the distal end of duodenoscopes, since the protruding forceps elevator tends to form a shadow zone which can impede the jets and sprays of treatment fluid and in practice negatively affect the effectiveness of the treatment.

An endoscopic cleaning device is described for example in document JP H03 280925 A.

There is therefore a need to perfect a device to support endoscopic instruments to be subjected to washing, sterilization and/or thermo disinfection treatment which can overcome at least one of the disadvantages of the state of the art.

In particular, one purpose of the present invention is to provide a device to support endoscopic instruments to be subjected to washing, sterilization and/or thermo disinfection treatment which overcomes at least one of the disadvantages of the state of the art.

Another purpose of the present invention is to provide a device to support articles to be subjected to washing and disinfection which allows to increase the efficiency of the washing treatment for endoscopes, and in particular duodenoscopes.

Another purpose of the invention is to provide a device to support articles which in particular allows an effective disinfection treatment of the distal end of duodenoscopes.

Another purpose of the present invention is to perfect a method for treating endoscopes, in particular duodenoscopes, which allows to perform a precise treatment of the distal end thereof.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, embodiments described here concern a support device to support at least one endoscopic instrument with an oblong conformation, to be subjected to a washing, sterilization and/or disinfection treatment in suitable washing machines; the support device comprises a frame provided with a support plane configured to receive and support the endoscopic instrument to be treated.

The support device can advantageously be used to support endoscopes, in particular duodenoscopes, even if it can be used to support articles of a different type in which it is necessary to carry out a targeted treatment on one portion thereof.

The support device comprises, mounted directly on board the frame, at least a positioning and localized treatment unit configured both to clamp and also to subject to a specific washing treatment, direct and localized, in a predefined position on the support plane, a target portion of the endoscopic instrument.

For example, in the case of a duodenoscope, the target portion corresponds to a distal terminal part in which there is the forceps elevator or fin, which allows to lift the tissues and to modify the angulation of the catheters and the guide wire, which, because of its complex shape, requires a specific and intensive washing and disinfection treatment.

The positioning and localized treatment unit comprises a clamping member configured to selectively clamp, in a predefined position on the support plane the target portion of the endoscopic instrument to be directly subjected to specific localized treatment, and a support member on which a nozzle to deliver a treatment fluid in a spray is mounted integrated, oriented toward the predefined position and the target portion positioned therein, in order to deliver, directly and in a localized manner, the treatment fluid onto the target portion, thus increasing the efficacy and the intensity of the treatment of the latter. The clamping member comprises an L-shaped elastic tongue for a releasable clamping of the target portion.

According to some embodiments, the support member comprises a shaped bracket provided with a support portion, on which the nozzle is mounted, and with a connection portion disposed between the support plane of the frame and the support portion, and configured to keep the support portion suspended at a determinate height above said support plane.

According to other embodiments, the support portion is inclined with respect to the support plane by a determinate angle of inclination, inclining the nozzle mounted thereon so that the delivery direction of the treatment fluid hits the target portion with a desired angle of incidence.

According to some embodiments, the elastic tongue comprises a first positioning arm transverse to the support plane and a second attachment arm that extends transversely from the first positioning arm and that is provided with a housing seating to receive and hold stably a portion of the endoscopic instrument to be treated adjacent to the target portion.

According to some embodiments, the positioning and localized treatment unit comprises a support base attached to the support plane.

According to some embodiments, the support base has a support surface above which at least part of the clamping member develops and a portion of the endoscopic instrument adjacent to the target portion is selectively positionable stably resting, between the support surface and the clamping member.

According to possible solutions, the support base connects the clamping member and the support member with respect to each other.

The present invention described here also concerns a method to perform a washing, sterilization and/or disinfection treatment of an endoscopic instrument in a treatment machine as defined in appended claim 12.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

### BRIEF DESCRIPTION OF THE DRAWINGS

These and other characteristics of the present invention will become apparent from the following description of some embodiments, given as a non-restrictive example with reference to the attached drawings wherein:

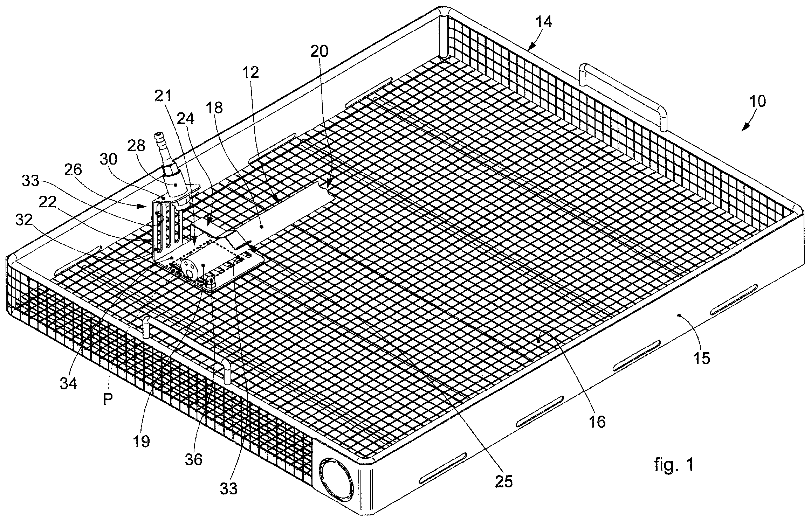

- fig. 1 is a schematic three-dimensional view of a support device according to embodiments described here;

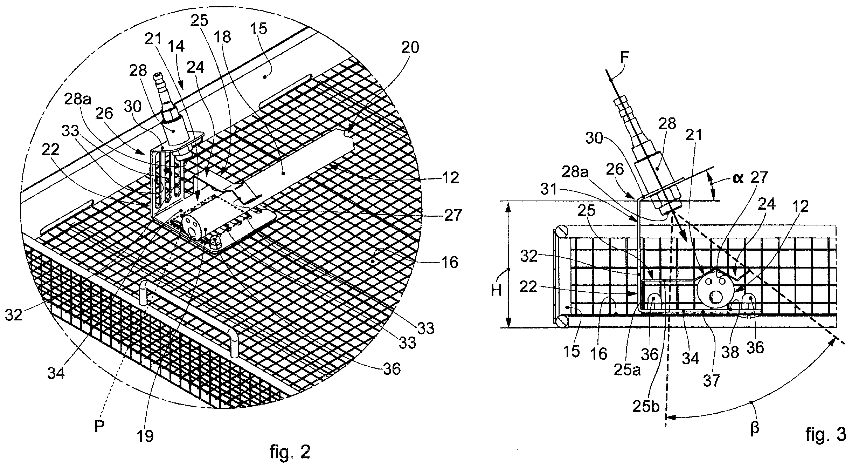

- fig. 2 is an enlarged detail of fig. 1;
- fig. 3 is a lateral view of the detail in fig. 2;
- fig. 4 is a view from below of a distal end of a duodenoscope with the forceps elevator in a lowered position;
- fig. 5 is a lateral view of the distal end of a duodenoscope with the forceps elevator in a lowered position;
- fig. 6 is a lateral view of the distal end of a duodenoscope with the forceps elevator in a raised position.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

Embodiments described here concern a support device, indicated in its entirety by the reference number 10, able to support at least one endoscopic instrument 12 to be subjected to washing, sterilization and/or disinfection treatment.

The support device 10 can be used in machines that perform the treatment of endoscopic instruments 12 by delivering a treatment liquid in the form of jets or sprays thereon.

In particular, the support device 10 is advantageously usable to support endoscopic instruments 12 having an oblong shape, in particular duodenoscopes, although it can be used to support articles of different types in which it is necessary to perform a targeted treatment on a target portion 21 thereof.

Other examples of endoscopes can be, for example, those used for the diagnosis or treatment of diseases affecting different organisms, including the lungs, stomach, esophagus, small intestine, biliary tract, pancreas or colon, or other.

The endoscopic instrument 12 comprises a tubular element 18 suitable to be inserted into the organisms to be analyzed, provided with two terminal parts, that is, a distal terminal part 19 and a proximal terminal part 20.

The term "distal terminal part" refers to the portion of the instrument which, during use, is farthest from the operator, while "proximal terminal part" refers to the portion of the instrument which, during use, is closest to the operator.

The proximal terminal part 20, during use, can be connected to a handling body, not shown, which allows the operator to grip the endoscope in order to insert it, and in particular the tubular element 18, into the parts of the organism to be analyzed.

The tubular element 18 can allow to deliver analysis liquids and for this purpose is fluidically connected to supply channels, not shown, in correspondence with the proximal terminal part 20.

The distal terminal part 19 can have, on each occasion, different shapes depending on the intended use of the endoscopic instrument 12.

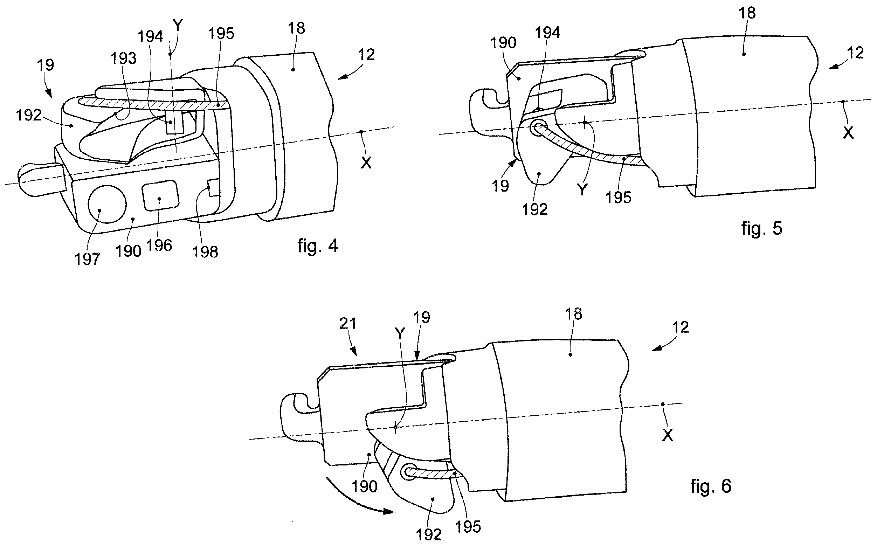

For example, in the case of a duodenoscope, partly shown in figs. 4-6, the distal terminal part 19 is provided with a support body 190 and a structure called forceps elevator 192, mobile with respect to the support body 190, which allows to modify the angle of the catheters and a guide wire: the distal terminal part 19 is also provided with a channel 193 associated therewith.

The forceps elevator 192 is pivoted to the support body 190 by a pin 194 which defines an axis of rotation Y, and is connected by a strand 195 to a hand piece, not shown, by means of which an operator can move it between a lowered position, in which it is aligned along a longitudinal axis X of the duodenoscope 12, and a raised position in which it is rotated around the axis of rotation Y in a direction transverse to the longitudinal axis X, for example by an angle of about 90°.

By longitudinal axis X we mean the axis along which the endoscopic instrument 12 develops when the endoscopic instrument 12 itself is in an elongated and distended condition.

Moreover, a lens or glass 196 and a light source 197, as well as the respective wiring, are generally present in the support body 190.

In the distal end 19 there can also be a nozzle 198, to deliver air, water, or other fluid.

According to some embodiments, the support body 190, the forceps elevator 192 and the other components of the distal terminal part 19 can be partly covered by a sheath, or a cap, not shown, possibly removable for disinfection, which nevertheless allows the movement of the forceps elevator 192 with respect to the support body 190.

During the use of the duodenoscope 12, this is generally inserted into the body of a patient with the forceps elevator 192 in the lowered position, that is, aligned with the longitudinal axis X, and is subsequently rotated to the desired inclination to modify the orientation of a guide wire or an instrument associated therewith.

During the use of the duodenoscope and during the rotation of the forceps elevator 192, the organic material may be deposited in the gap between the latter and the support body 190, or inside the channel 193, making the disinfection of the distal terminal part 19 complicated. The distal terminal part 19 thus defines the target portion 21 of the duodendoscope 12 which requires a specific and intensive treatment to guarantee washing and disinfection at a level sufficient to prevent possible contamination.

The support device 10 comprises a frame 14 having a support plane 16 on which, during use, at least one endoscopic instrument 12 to be treated is positioned, possibly delimited by lateral walls 15.

According to some embodiments, the frame 14 has a substantially rectangular shape although it cannot be excluded that, in other embodiments, the frame 14 can have a different shape, for example circular or polygonal.

According to possible embodiments, the support plane 16 can be substantially flat, as in the solution shown in the attached drawings, or it can have conformations having portions that develop on inclined or offset planes according to specific application requirements.

According to some embodiments, the frame 14, or at least part of it, is the reticular type, that is, configured in the form of a net, defined by meshes obtained by arms, bars or similar interconnected oblong elements. This reticular conformation allows the passage of the treatment fluids and/or liquids and their drainage when necessary.

According to some embodiments, it can be provided that at least the support plane 16 is the reticular type, so as to allow the drainage of the fluids and/or liquids.

In accordance with possible solutions, the frame 14 can advantageously be made of a material suitable to support conditions of washing, sterilization and/or thermo disinfection treatment, for example a metal material, for example stainless steel or aluminum, or a polymer material, for example polyethylene or polyamide.

The support device 10 comprises at least one localized positioning and treatment unit 22, mounted directly on board the frame 14, and configured to clamp the target portion 21 of the endoscope instrument 12 to be treated and subject it to specific localized direct treatment to in a predefined position P on the support plane 16.

According to some embodiments, the localized positioning and treatment unit 22 is configured to position the target portion 21 of the endoscope 12 to be treated, in this specific case a portion of the distal terminal part 19 of the endoscope 12, in the predefined position P and/or with a defined orientation.

According to some embodiments, the localized positioning and treatment unit 22 is attached to the support plane 16.

According to some embodiments, the targeted positioning and treatment unit 22 is attached to the support plane 16 with removable attachment members 36, so as to be able to move it on each occasion to the most suitable position according to the requirements of the treatment process and/or type of treatment machine used.

The targeted positioning and treatment unit 22 comprises a clamping member 24 configured to selectively clamp, in the predefined position P on the support plane 16, the target portion 21 of the endoscopic instrument 12 to be subjected directly to a specific localized treatment.

The targeted positioning and treatment unit 22 also comprises a support member 26 on which a treatment fluid delivery nozzle 28 is mounted, oriented toward the predefined position P, so as to deliver, directly and in a localized manner, the treatment fluid onto the target portion 21 held by the clamping member 24.

According to some embodiments, the treatment fluid is in particular a liquid, for example water mixed with possible chemical detergents or sanitizers.

The nozzle 28 is in particular configured to deliver the treatment liquid onto the target portion in the form of jets, or sprays, with a pressure suitable to remove any residues of organic material and to ensure suitable disinfection.

The clamping member 24 comprises an elastic tongue 25 in order to releasably clamp the target portion 21.

The elastic tongue 25 is suitable to exert a thrust on the tubular body 18 of the endoscope 12, pressing it toward the support plane 16.

The elastic tongue 25 is L-shaped, with a first positioning arm 25a disposed on a plane transverse to the support plane 16 and a second attachment arm 25b which extends transversely from the first positioning arm 25a.

According to some embodiments, the second attachment arm 25b extends substantially parallel to the support plane 16.

According to some embodiments, the second positioning arm 25a is provided with a housing seating 27 to receive and stably hold a portion of the endoscopic instrument 12, in particular a portion adjacent to the target portion 21 so as not to interfere with the delivery of the treatment fluid delivered by the nozzle 28.

According to possible solutions, the second attachment arm 25b can be bent in such a way as to define the housing seating 27 on the side facing toward the support plane 16.

According to some embodiments, the housing seating 27 can hold the endoscopic instrument 12 in position, preventing it from rotating around its own longitudinal axis, so as to maintain a correct orientation of the target portion 21 with respect to the nozzle 28 during the treatment process.

According to some embodiments, not shown, the clamping member 24 can comprise one or more holding elements associated with the support plane 16 and defining one or more housing seatings suitable to receive and hold the endoscope 12 in a pre-defined position and with a pre-defined orientation in order to direct the target portion 21 toward the nozzle 28.

According to some embodiments, the clamping member 24 is configured to position the target portion 21 in a delivery direction F of the nozzle 28, so that the treatment fluid delivered by the latter directly impacts thereon.

According to some embodiments, during use, the nozzle 28 can be connected in a known manner to devices for supplying treatment fluids or liquids provided in the treatment machine.

According to possible embodiments, the support member 26 comprises a shaped bracket 31 provided with a support portion 30 on which the nozzle 28 is mounted, and with a connection portion 32 disposed between the support plane 16 and the support portion 30, configured to keep the support portion 30 at a certain height H above the support plane 16.

According to some embodiments, the support portion 30 is inclined by a determinate angle of inclination α with respect to the support plane 16, for example comprised between 20° and 40°, preferably between 25° and 35°.

According to some embodiments, the height H and/or the angle of inclination α can be chosen, for example, according to the sizes of the endoscopic instrument 12 to be treated and therefore to the area of the predefined position P needed, the type of nozzle 28 used, the type of treatment, the treatment fluid, or other.

In accordance with possible embodiments, the nozzle 28 has a delivery aperture 28a configured to deliver the treatment fluid in the delivery direction F.

According to some embodiments, the delivery direction F hits the support plane 16 with an angle comprised between about 50° and 70°, preferably between about 55° and about 65°.

According to some embodiments, the delivery aperture 28a can be configured to deliver the treatment fluid with a delivery cone having an angular amplitude β between about 40° and about 60°, preferably between about 45° and about 55° so as to hit a large part of the target portion 21.

According to some embodiments, the support portion 30 and the connection portion 32 are stably connected to each other and/or made in a single body.

According to other embodiments, it can be provided that the support portion 30 can be connected to the connection portion 32 by means of hinging means which allow a relative rotation movement between them, allowing to modify the inclination of the nozzle 28 according to needs.

According to some embodiments, the targeted positioning and treatment unit 22 comprises a support base 34 configured to be disposed on the support plane 16 and attached thereto.

According to some embodiments, the support base 34 is provided with a lower surface 37 disposed, during use, on the support plane 16, and a support surface 38 opposite to it, on which, during use, the endoscope 12 to be treated can be positioned.

According to some embodiments, the support base 34 can be connected to the support plane 16 by means of attachment members 36 of a removable type, for example screws, bolts, pins, or suchlike.

In this way, the localized positioning and treatment unit 22 can be located in any position whatsoever on the support plane 16, for example as a function of the endoscopic instrument 12 to be treated, the type of treatment machine to be used and the disposition of the devices to supply the treatment fluid therein, so as to be able to connect them quickly to the nozzle 28.

According to some embodiments, the clamping member 24 develops at least partly above the support surface 38 and a portion of the endoscopic instrument 12, adjacent to the target portion 21, can be selectively positioned stably resting between the surface support 38 and the clamping member 24.

According to some embodiments, the support base 34 connects the clamping member 24 and the support member 26 with each other.

According to some embodiments, the clamping member 24 and the support member 26 can be made in a single body with the support base 34.

According to some embodiments, the support member 26 and the support base 34 can be made of a single C-shaped body.

According to other embodiments, the clamping member 24 and the support member 26 can be made as separate bodies and autonomously mounted on the support plane 16.

According to some embodiments, the support member 24, or the connection portion 32 and/or the support base 34 can be provided with through holes or slits 33, suitable to allow the passage of treatment fluids or liquids and/or their drainage through them.

According to a possible solution, the predefined position P can be delimited by the support surface 38 or by a central region thereof.

According to some embodiments, the clamping member 24 develops above a peripheral band of the support surface 38 in such a way that the target portion 21 is positioned in the central region, which frames the predefined position P of the support surface 38.

According to some embodiments, the nozzle 28 can be conformed in such a way as to deliver the treatment fluid over an area having an extension substantially equal to the central region of the support base 34 which frames the predefined portion P and not affected by the clamping member 24.

The present invention described here also concerns a method to perform a washing, sterilization and/or disinfection treatment of an endoscopic instrument 12 in a treatment machine.

The method provides to perform a specific localized direct treatment of the target portion 21 of the endoscopic instrument 12 automatically and directly on board the support device 10 by means of the localized positioning and treatment unit 22.

In the case of a duodendoscopic instrument 12, the method provides to rotate the forceps elevator 192 in a raised position, rotated by about 90° with respect to the longitudinal axis X of the duodenoscope 12, so as to make available a free space between the support body 190 and the forceps elevator 192, and to subsequently position the duodenoscope 12 in the localized positioning and treatment unit 22.

According to some embodiments, the duodenoscope 12 can be positioned so as to direct the forceps elevator 192 to the opposite side of the nozzle 28 and deliver the treatment liquid into the free space between the support body 190 and the rotated forceps elevator 192.

It is clear that modifications and/or additions of parts can be made to the support device and treatment method as described heretofore, without departing from the field and scope of the present invention.

It is also clear that, although the present invention has been described with reference to some specific examples, a person of skill in the art shall certainly be able to achieve many other forms of support device and treatment method, having the characteristics as set forth in the claims and hence all coming within the field of protection defined thereby.

## Claims

1. Support device to support at least one endoscopic instrument (12) with an oblong conformation, to be subjected to a washing, sterilization and/or disinfection treatment, said support device comprising a frame (14) with a support plane (16) for said at least one endoscopic instrument (12), wherein said support device comprises, mounted directly on board of said frame (14), at least a positioning and localized treatment unit (22) configured both to clamp and also to subject to a specific washing treatment, direct and localized, in a predefined position (P) on said support plane (16), a target portion (21) of said endoscopic instrument (12), said positioning and localized treatment unit (22) comprising a support member (26) on which a nozzle (28) to deliver a treatment fluid in a spray is mounted integrated, oriented toward said predefined position (P) and said target portion (21) positioned therein, in order to deliver, directly and in a localized manner, the treatment fluid onto said target portion (21),
**characterized in that** said positioning and localized treatment unit (22) comprises:
- a clamping member (24) configured to selectively clamp, in a predefined position (P) on said support plane (16), a target portion (21) of said endoscopic instrument (12) to be directly subjected to specific localized treatment, said clamping member (24) comprising an L-shaped elastic tongue (25) for a releasable clamping of said
target portion (21).

2. Support device as in claim 1, **characterized in that** said support member (26) comprises a shaped bracket (31) provided with a support portion (30), on which the nozzle (28) is mounted, and with a connection portion (32) disposed between the support plane (16) of the frame (14) and said support portion (30), and configured to keep said support portion (30) suspended at a determinate height (H) above said support plane (16).

3. Support device as in claim 2, **characterized in that** said support portion (30) is inclined with respect to said support plane (16) by an angle of inclination (α) comprised between 20° and 40°.

4. Support device as in claim 1, **characterized in that** said elastic tongue (25) comprises a first positioning arm (25a) transverse to the support plane (16) and a second attachment arm (25b) that extends transversely from the first positioning arm (25a) and that is provided with a housing seating (27) to receive and hold stably a portion of said endoscopic instrument (12) adjacent to said target portion (21).

5. Support device as in any claim hereinbefore, **characterized in that** said positioning and localized treatment unit (22) comprises a support base (34) attached to said support plane (16).

6. Support device as in claim 5, **characterized in that** said support base (34) has a support surface (38) above which at least part of said clamping member (24) develops, a portion of said endoscopic instrument (12) adjacent to said target portion (21) being selectively positionable stably resting between said support surface (38) and said clamping member (24).

7. Support device as in claim 5 or 6, **characterized in that** said support base (34) connects said clamping member (24) and said support member (26) with respect to each other.

8. Support device as in claim 6 or 7, **characterized in that** said clamping member (24) develops above a peripheral band of said support surface (38), so that said target portion (21) is positioned in a central area of said support surface (38) which frames said predefined position (P), and said nozzle (28) is configured to deliver the treatment fluid on said central area.

9. Support device as in claim 1, **characterized in that** said nozzle (28) is provided with a delivery aperture (28a) configured to deliver the treatment fluid with a delivery cone having an angular amplitude between about 40° and about 60°, so as to hit a large part of said target portion (21).

10. Support device as in claim 1, **characterized in that** said clamping member (24) and said support member (26) are integrated in a single body or assembly, directly mounted on said support plane (16), or said clamping member (24) and said support member (26) are made as separate bodies, autonomously mounted on said support plane (16).

11. Support device as in any claim hereinbefore, **characterized in that** said endoscopic instrument (12) is a duodenoscope (12) and said target portion (21) is a distal terminal part (19) of said duodenoscope (12) comprising a support body (190) and a forceps elevator (192) rotatable to a raised position rotated by about 90° with respect to said support body (190) and a longitudinal axis (X) of said duodenoscope (12).

12. Method to perform a washing, sterilization and/or disinfection treatment of an endoscopic instrument (12) in a treatment machine, **characterized in that** said method provides to perform a specific localized direct treatment of a target portion (21) of said endoscopic instrument (12) automatically and directly on board of a support device (10) as in any claim hereinbefore, by means of at least the positioning and localized treatment unit (22) mounted directly on board of the frame (14) of said support device (10) and which is used to clamp and subject said target portion (21) to a specific localized direct treatment, in the predefined position (P) on the support plane (16) of said frame (14).

13. Method as in claim 12, **characterized in that** the specific localized direct treatment comprises the delivery of a treatment fluid in the form of jets, or sprays, on said target portion (21) by means of the nozzle (28) provided in said positioning and localized treatment unit (22).

14. Method as in claim 13, **characterized in that** said endoscopic instrument (12) is a duodenoscope (12) and said target portion (21) is a distal terminal part (19) of said duodenoscope (12), wherein said method provides to rotate a forceps elevator (192) located in said distal terminal part (19) to a raised position, rotated by about 90° with respect to a longitudinal axis (X) and to a support body (190) of said duodenoscope (12), and subsequently to position said distal terminal part (19) in said positioning and localized treatment unit (22), directing said forceps elevator (192) in the opposite direction to said nozzle (28) so as to deliver said treatment fluid from said nozzle (28) in a free space between said support body (190) and said forceps elevator (192) made available by the rotation of the latter.

## Patentansprüche

1. Haltevorrichtung zum Halten zumindest eines endoskopischen Instruments (12) mit einer länglichen Form, das einer Wasch-, Sterilisations- und/oder Desinfektionsbehandlung unterzogen werden soll, wobei die Haltevorrichtung einen Rahmen (14) mit einer Halteebene (16) für das zumindest eine endoskopische Instrument (12) aufweist, wobei die Haltevorrichtung aufweist, direkt an Bord des Rahmens (14) angebracht, zumindest eine Positionierungs- und lokalisierte Behandlungseinheit (22), die konfiguriert ist sowohl zum Festklemmen als auch zum Unterziehen einer spezifischen Waschbehandlung, direkt und lokalisiert an einer vordefinierten Position (P) auf der Halteebene (16), eines Zielabschnitts (21) des endoskopischen Instruments (12), wobei die Positionierungs- und lokalisierte Behandlungseinheit (22) ein Halteelement (26) aufweist, an dem eine Düse (28) zum Zuführen eines Behandlungsfluid als ein Sprühnebel integriert angebracht ist, die auf die vordefinierte Position (P) und den in dieser positionierten Zielabschnitt (21) ausgerichtet ist, um das Behandlungsfluid direkt und lokalisiert auf den Zielabschnitt (21) zuzuführen, **dadurch gekennzeichnet, dass** die Positionierungs- und lokalisierte Behandlungseinheit (22) aufweist:
- ein Klemmelement (24), das konfiguriert ist, um einen Zielabschnitt (21) des endoskopischen Instruments (12), der direkt einer spezifischen lokalisierten Behandlung unterzogen werden soll, selektiv in einer vordefinierten Position (P) auf der Halteebene (16) festzuklemmen, wobei das Klemmelement (24) eine L-förmige elastische Zunge (25) zum lösbaren Festklemmen des Abschnitts (21) aufweist.

2. Haltevorrichtung gemäß Anspruch 1, **dadurch gekennzeichnet, dass** das Halteelement (26) eine geformte Halterung (31) aufweist, die mit einem Halteabschnitt (30), an dem die Düse (28) angebracht ist, und mit einem Verbindungsabschnitt (32) versehen ist, der zwischen der Halteebene (16) des Rahmens (14) und dem Halteabschnitt (30) angeordnet ist und konfiguriert ist, um den Halteabschnitt (30) in einer bestimmten Höhe (H) über der Halteebene (16) freiaufgehängt zu halten.

3. Haltevorrichtung gemäß Anspruch 2, **dadurch gekennzeichnet, dass** der Halteabschnitt (30) in Bezug auf die Halteebene (16) um einen Neigungswinkel (α), der zwischen 20° und 40° liegt, geneigt ist.

4. Haltevorrichtung gemäß Anspruch 1, **dadurch gekennzeichnet, dass** die elastische Zunge (25) einen ersten Positionierungsarm (25a) quer zur Halteebene (16) und einen zweiten Befestigungsarm (25b) aufweist, der sich vom ersten Positionierungsarm (25a) aus quer erstreckt und der mit einem Aufnahmesitz (27) versehen ist, um einen Abschnitt des endoskopischen Instruments (12) benachbart zu dem Zielabschnitt (21) aufzunehmen und stabil zu halten.

5. Haltevorrichtung gemäß irgendeinem vorstehenden Anspruch, **dadurch gekennzeichnet, dass** die Positionierungs- und lokalisierte Behandlungseinheit (22) eine Haltebasis (34) aufweist, die an der Halteebene (16) angebracht ist.

6. Haltevorrichtung gemäß Anspruch 5, **dadurch gekennzeichnet, dass** die Haltebasis (34) eine Haltefläche (38) aufweist, über der sich zumindest ein Teil des Klemmelements (24) erstreckt, wobei ein Abschnitt des endoskopischen Instruments (12) benachbart zu dem Zielabschnitt (21) wahlweise stabil ruhend zwischen der Haltefläche (38) und dem Klemmelement (24) positioniert werden kann.

7. Haltevorrichtung gemäß Anspruch 5 oder 6, **dadurch gekennzeichnet, dass** die Haltebasis (34) das Klemmelement (24) und das Halteelement (26) miteinander verbindet.

8. Haltevorrichtung gemäß Anspruch 6 oder 7, **dadurch gekennzeichnet, dass** sich das Klemmelement (24) über einem Umfangsband der Haltefläche (38) erstreckt, so dass der Zielabschnitt (21) in einem zentralen Bereich der Haltefläche (38) positioniert ist, der die vordefinierte Position (P) einrahmt, und wobei die Düse (28) so konfiguriert ist, dass sie das Behandlungsfluid auf den zentralen Bereich zuführt.

9. Haltevorrichtung gemäß Anspruch 1, **dadurch gekennzeichnet, dass** die Düse (28) mit einer Zuführöffnung (28a) versehen ist, die so konfiguriert ist, dass sie das Behandlungsfluid mit einem Zuführkegel, der eine Winkelgröße zwischen etwa 40° und etwa 60° hat, zuführt, um einen großen Teil des Zielabschnitts (21) zu treffen.

10. Haltevorrichtung gemäß Anspruch 1, **dadurch gekennzeichnet, dass** das Klemmelement (24) und das Halteelement (26) in einem einzigen Körper oder einer einzigen Baugruppe integriert sind, der/die direkt auf der Halteebene (16) angebracht ist, oder dass das Klemmelement (24) und das Halteelement (26) als separate Körper ausgebildet sind, die unabhängig auf der Halteebene (16) angebracht sind.

11. Haltevorrichtung gemäß irgendeinem vorstehenden Anspruch, **dadurch gekennzeichnet, dass** das endoskopische Instrument (12) ein Duodenoskop (12) ist und der Zielabschnitt (21) ein distaler Endteil (19) des Duodenoskops (12) ist, der einen Haltekörper (190) und einen Zangenheber (192) aufweist, der in eine angehoben-Position gedreht werden kann, die um etwa 90° in Bezug auf den Haltekörper (190) und eine Längsachse (X) des Duodenoskops (12) gedreht ist.

12. Verfahren zum Durchführen einer Wasch-, Sterilisations- und/oder Desinfektionsbehandlung eines endoskopischen Instruments (12) in einer Behandlungsmaschine, **dadurch gekennzeichnet, dass** das Verfahren bereitstellt, eine spezifische lokalisierte direkte Behandlung eines Zielabschnitts (21) des endoskopischen Instruments (12) automatisch und direkt an Bord einer Haltevorrichtung (10) gemäß irgendeinem vorstehenden Anspruch durchzuführen mittels zumindest der Positionierungs- und lokalisierten Behandlungseinheit (22), die direkt an Bord des Rahmens (14) der Haltevorrichtung (10) angebracht ist und welche dazu dient, den Zielabschnitt (21) in der vordefinierten Position (P) auf der Halteebene (16) des Rahmens (14) festzuklemmen und einer spezifischen lokalisierten direkten Behandlung zu unterziehen.

13. Verfahren gemäß Anspruch 12, **dadurch gekennzeichnet, dass** die spezifische lokalisierte direkt Behandlung die Zuführung eines Behandlungsfluid in Form von Strahlen oder Sprühnebeln auf den Zielabschnitt (21) mittels der Düse (28) aufweist, die in der Positionierungs- und lokalisierten Behandlungseinheit (22) bereitgestellt ist.

14. Verfahren gemäß Anspruch 13, **dadurch gekennzeichnet, dass** das endoskopische Instrument (12) ein Duodenoskop (12) ist und der Zielabschnitt (21) ein distaler Endteil (19) des Duodenoskops (12) ist, wobei das Verfahren bereitstellt, einen Zangenheber (192), der sich in dem distalen Endteil (19) befindet, in eine angehoben-Position zu drehen, die um etwa 90° in Bezug auf eine Längsachse (X) und auf einen Haltekörper (190) des Duodenoskops (12) gedreht ist, und anschließend den distalen Endteil (19) in der Positionierungs- und lokalisierten Behandlungseinheit (22) zu positionieren, wodurch der Zangenheber (192) in die entgegengesetzte Richtung zur Düse (28) ausgerichtet wird, um das Behandlungsfluid aus der Düse (28) in einen freien Raum zwischen dem Haltekörper (190) und dem Zangenheber (192) zuzuführen, der durch die Drehung des Letzteren breitgestellt wird.

## Revendications

1. Dispositif porteur pour supporter au moins un instrument endoscopique (12) de conformation oblongue, à soumettre à un traitement de lavage, de stérilisation et/ou de désinfection, ledit dispositif porteur comprenant un cadre (14) avec un plan de support (16) pour ledit au moins un instrument endoscopique (12), dans lequel ledit dispositif porteur comprend, monté directement sur ledit cadre (14), au moins une unité de positionnement et de traitement localisé (22) configurée à la fois pour serrer et également pour soumettre à un traitement de lavage spécifique, direct et localisé, dans une position prédéfinie (P) sur ledit plan de support (16), une partie cible (21) dudit instrument endoscopique (12), ladite unité de positionnement et de traitement localisé (22) comprenant un élément porteur (26) sur lequel une buse (28) pour distribuer un fluide de traitement sous forme de pulvérisation est montée d'un seul bloc, orientée vers ladite position prédéfinie (P) et ladite partie cible (21) positionnée dans celle-ci, afin de distribuer, directement et de manière localisée, le fluide de traitement sur ladite partie cible (21),
**caractérisé en ce que** ladite unité de positionnement et de traitement localisé (22) comprend :
- un élément de serrage (24) configuré pour serrer sélectivement, dans une position prédéfinie (P) sur ledit plan de support (16), une partie cible (21) dudit instrument endoscopique (12) à soumettre directement à un traitement localisé spécifique, ledit élément de serrage (24) comprenant une languette élastique en L (25) pour un serrage libérable de ladite partie cible (21).

2. Dispositif porteur selon la revendication 1, **caractérisé en ce que** ledit élément porteur (26) comprend un support profilé (31) muni d'une partie porteuse (30), sur laquelle est montée la buse (28), et d'une partie de raccordement (32) disposée entre le plan de support (16) du cadre (14) et ladite partie porteuse (30), et configuré pour maintenir ladite partie porteuse (30) suspendue à une hauteur déterminée (H) au-dessus dudit plan de support (16).

3. Dispositif porteur selon la revendication 2, **caractérisé en ce que** ladite partie porteuse (30) est inclinée par rapport audit plan de support (16) d'un angle d'inclinaison (α) compris entre 20° et 40°.

4. Dispositif porteur selon la revendication 1, **caractérisé en ce que** ladite languette élastique (25) comprend un premier bras de positionnement (25a) transversal au plan de support (16) et un second bras de fixation (25b) qui s'étend transversalement depuis le premier bras de positionnement (25a) et qui est muni d'un siège de logement (27) pour recevoir et maintenir de manière stable une partie dudit instrument endoscopique (12) adjacente à ladite partie cible (21).

5. Dispositif porteur selon une quelconque revendication précédente, **caractérisé en ce que** ladite unité de positionnement et de traitement localisé (22) comprend une base porteuse (34) fixée audit plan de support (16).

6. Dispositif porteur selon la revendication 5, **caractérisé en ce que** ladite base porteuse (34) présente une surface porteuse (38) au-dessus de laquelle se développe au moins une partie dudit élément de serrage (24), une partie dudit instrument endoscopique (12) adjacente à ladite partie cible (21) pouvant être positionnée sélectivement en appui stable entre ladite surface porteuse (38) et ledit élément de serrage (24).

7. Dispositif porteur selon la revendication 5 ou 6, **caractérisé en ce que** ladite base porteuse (34) relie ledit élément de serrage (24) et ledit élément porteur (26) l'un à l'autre.

8. Dispositif porteur selon la revendication 6 ou 7, **caractérisé en ce que** ledit élément de serrage (24) se développe au-dessus d'une bande périphérique de ladite surface porteuse (38), de sorte que ladite partie cible (21) soit positionnée dans une zone centrale de ladite surface porteuse (38) qui encadre ladite position prédéfinie (P), et ladite buse (28) est configurée pour distribuer le fluide de traitement sur ladite zone centrale.

9. Dispositif porteur selon la revendication 1, **caractérisé en ce que** ladite buse (28) est munie d'une ouverture de distribution (28a) configurée pour distribuer le fluide de traitement avec un cône de distribution présentant une amplitude angulaire comprise entre environ 40° et environ 60°, de manière à atteindre une grande partie de ladite partie cible (21).

10. Dispositif porteur selon la revendication 1, **caractérisé en ce que** ledit élément de serrage (24) et ledit élément porteur (26) sont intégrés en un seul corps ou ensemble, directement monté sur ledit plan de support (16), ou ledit élément de serrage (24) et ledit élément porteur (26) se présentent sous forme de corps séparés, montés de manière autonome sur ledit plan de support (16).

11. Dispositif porteur selon une quelconque revendication précédente, **caractérisé en ce que** ledit instrument endoscopique (12) est un duodénoscope (12) et ladite partie cible (21) est une partie terminale distale (19) dudit duodénoscope (12) comprenant un corps porteur (190) et un élévateur à pinces (192) pouvant tourner vers une position relevée tournée d'environ 90° par rapport audit corps porteur (190) et un axe longitudinal (X) dudit duodénoscope (12).

12. Procédé de réalisation d'un traitement de lavage, de stérilisation et/ou de désinfection d'un instrument endoscopique (12) dans une machine de traitement, **caractérisé en ce que** ledit procédé comprend la réalisation d'un traitement direct localisé spécifique d'une partie cible (21) dudit instrument endoscopique (12) automatiquement et directement sur un dispositif porteur (10) selon une quelconque revendication précédente, au moyen d'au moins l'unité de positionnement et de traitement localisé (22) montée directement sur le cadre (14) dudit dispositif porteur (10) et qui est utilisée pour serrer et soumettre ladite partie cible (21) à un traitement direct localisé spécifique, dans la position prédéfinie (P) sur le plan de support (16) dudit cadre (14).

13. Procédé selon la revendication 12, **caractérisé en ce que** le traitement direct localisé spécifique comprend la distribution d'un fluide de traitement sous forme de jets, ou de pulvérisations, sur ladite partie cible (21) au moyen de la buse (28) disposée dans ladite unité de positionnement et de traitement localisé (22).

14. Procédé selon la revendication 13, **caractérisé en ce que** ledit instrument endoscopique (12) est un duodénoscope (12) et ladite partie cible (21) est une partie terminale distale (19) dudit duodénoscope (12), dans lequel ledit procédé comprend la rotation d'un élévateur à pinces (192) situé dans ladite partie terminale distale (19) vers une position relevée, tournée d'environ 90° par rapport à un axe longitudinal (X) et à un corps porteur (190) dudit duodénoscope (12), puis le positionnement de ladite partie terminale distale (19) dans ladite unité de positionnement et de traitement localisé (22), en dirigeant ledit élévateur à pinces (192) dans la direction opposée à ladite buse (28) de manière à distribuer ledit fluide de traitement à partir de ladite buse (28) dans un espace libre entre ledit corps porteur (190) et ledit élévateur à pinces (192) rendu accessible par la rotation de ce dernier.
